# EUROPEAN PATENT APPLICATION

(11) **EP 4 545 175 A1**
(43) Date of publication of application: **30.04.2025**
(21) Application number: 24205569.7
(22) Date of filing: 09.10.2024
(51) Int. Cl.: B01J 23/78, B01J 23/83, B01J 35/45, B01J 35/54, B01J 35/61, B01J 35/63, B01J 35/30, B01J 37/02, B01J 37/03, B01J 37/08, B01J 37/10, B01J 37/18, C07C 1/12, C07C 9/06, C07C 11/04

(54) **PROCESS FOR CONVERSION OF CARBON DIOXIDE AND HYDROGEN INTO ETHANE AND ETHYLENE**

(30) Priority: 23.10.2023 LU 505337
(71) Applicant: Kemijski Institut, 1000 Ljubljana (SI)
(72) Inventor: DJINOVIC, Petar, 1000 Ljubljana (SI); LORBER, Kristjan, 1000 Ljubljana (SI); NOVAK, Tusar Natasa, 1000 Ljubljana (SI); ZABUKOVEC, Logar Natasa, 1000 Ljubljana (SI)
(74) Representative: Weickmann & Weickmann PartmbB

(57) **Abstract**

This invention relates to a process of direct hydrogenation of carbon dioxide gas using molecular hydrogen to produce methane, carbon monoxide, ethane or ethylene. The carbon based selectivity for C2 products (ethane and ethylene) reaches up to 15% and productivity of 0.24 gC₂/g_{catalyst}*h, if compared to existing C2 selectivity of 3% and productivity of 0.0001 gC₂/g_{catalyst}*h. In particular, the invention requires a composition comprised of nickel nanoparticles deposited on cerium oxide nanorod powder support to act as a catalyst for the chemical reaction. The performance of the catalyst for C2 production is improved by alkali doping, that is modification with small amounts of potassium or caesium.

## Description

### FIELD OF INVENTION

This invention relates to a process of direct catalytic hydrogenation of carbon dioxide gas using gaseous hydrogen into a gas stream containing ethane and ethylene. In particular, the invention requires a composition comprised of nickel nanoparticles deposited on cerium oxide powder support to act as a catalyst for the chemical reaction. The performance of the catalyst is improved by alkali doping, that is modification with small amounts of e.g. potassium or caesium.

### BACKGROUND OF INVENTION

Hydrogenation of carbon dioxide to produce carbon monoxide, methanol or methane is a viable approach for valorization of hydrogen and carbon dioxide into chemicals. The scientific literature and patent applications are abundant for these chemical conversions. However, realization of carbon coupling reactions to produce C-C bonds directly from carbon dioxide is limited because of kinetic and thermodynamic restrictions. The catalysts that enable formation of multi-carbon reaction products require presence of CO in the feed and iron or cobalt based catalysts. This is known as the Fischer-Tropsch chemistry. However, when typical Fischer-Tropsch catalysts run under the H₂/CO₂ feed, methane is the main product and C2 and C3 selectivity reach 3% at best [https://doi.org/10.1021/ef900275m].

Ethane is interesting from the aspect of its further dehydrogenation to ethylene, which is a main building block for polyethylene plastics and other value-added chemicals.

### SUMMARY OF INVENTION

We have discovered that a catalytic reaction between CO₂ and H₂ using selected catalyst produces methane, carbon monoxide, ethane and ethylene. The C2 selectivity reaches up to 15%.

The catalyst for use according to the invention is a heterogeneous catalyst comprising nickel nanoparticles on a solid support, and further comprising at least one alkali metal supported on the solid support.

In particular embodiments, cerium dioxide (ceria, CeO₂) powder acts as a catalyst support, and is synthesized using a hydrothermal method, so the particles have a rod-like shape.

The ceria support is decorated with nickel using precipitation and aging of nickel nitrate in alkaline solution achieved by aqueous ammonia solution. Finally, the material is calcined in a chamber furnace at 500°C in air.

By contacting a gas flow comprising of CO₂, H₂ and N₂ over the catalyst, the catalytic reaction which occurs between 250 and 360°C produces CO, methane, ethane and ethylene.

The catalyst without alkali modification yields very low selectivity (total up to 2.7%) of ethane and ethylene.

Ethane selectivity can be favored (total up to 10%) by modification of the catalyst with alkalis in particular potassium, whereas ethylene selectivity is enabled by modification with caesium.

### DETAILED DESCRIPTION OF INVENTION

The present invention enables catalytic conversion of CO₂ and hydrogen into ethane and ethylene containing gas mixture, via a hydrogenation reaction using a nickel-based catalyst supported on solid material, (preferably cerium dioxide) and modified with alkali cations such as potassium or caesium.

Different powdered solid materials can be used for supporting the nickel, such as alumina, silica, titania or similar. However, the support material most preferred for use in the present innovation is cerium dioxide (CeO₂). Among several possible shapes of cerium dioxide crystals, nanorod shape is preferred. The term "nanorod" refers to an elongated three-dimensional structure in which the expansion in one dimension is greater than in the other two dimensions, but in which all dimensions are in the nano range (1-500 nm). Sizes and shapes of structures the nanometer range can be measured e.g., by atomic force microscopy (AFM), Transmission electron microscopy (TEM), or dynamic light scattering (DLS). Typically, nanorods are rods, pillars, or columns with a lateral dimension on the order of tens of nanometers. Their aspect ratio (length divided by width) is typically on the order of 50 or smaller, e.g. 10-25. Such ceria morphology is providing exceptionally high ethane and ethylene selectivity and was found superior to other support materials. The surface area of the ceria support material is generally at least 5 m²/g. Preferably, ceria supports having high specific surface area (e.g. 50-100 m²/g) are most effective for the purpose of this invention. The surface area is measured by a conventional nitrogen sorption method and application of BET theory. The support material in this invention can be described as material containing interparticle porosity with a pore volume between 0.05 and 0.6 cm³/g. Preferably the solid support contains pores with diameters in a range between 2 and 50 nm, in particular wherein the pore volume is in a range of about 0.05 to 0.6 cm³/g based on the total weight of the solid support. For determining pore diameter and pore volume, widely used characterization method, such as N₂ physisorption is used.

The ceria support for the catalytic application in this invention can be synthesized in various shapes of crystals. By doing so, we are changing the morphology and chemical reactivity of support. Ceria support that is synthesized in the shape of nanorods is preferred. The individual nanorods have a diameter of about 10-20 nm and length between 100 and 500 nm.

The ceria support may be synthesized by any known method, such as precipitation or combustion synthesis. A preferred method for ceria nanorod synthesis is a precipitation process where cerium nitrate hexahydrate salt is dissolved in ultrapure water. The pH value of the solution is increased by 10 M aqueous NaOH solution while stirring on a magnetic stirrer at 400-600 rpm. After precipitation, the suspension is aged/stirred for 30 minutes at room temperature and transferred to Teflon lined stainless steel autoclaves for hydrothermal aging.

The hydrothermal aging is performed by placing the autoclaves into laboratory drier, which is preheated to desired temperature. Typical temperatures are between 50 and 200°C. The preferred temperature is 100°C, as this will yield the desired nanorod shape of the ceria crystals. The hydrothermal aging time is important for ensuring sufficient time to grow the nanorod crystals. Aging time can vary between 5 and 48 hours. The preferred aging time is 24 hours.

After hydrothermal aging, the autoclaves are taken out of the drier and cooled to ambient temperature by quenching in cold water. This usually takes about 15 minutes. Once the autoclaves have attained ambient temperature, the suspension is washed and centrifuged 3 minutes at 7500 rpm to separate the precipitate from the supernatant three times with ultrapure water and one time with absolute ethanol. The washed precursor is placed on the Petri dish and dried in the laboratory drier in air at 60°C. The preferred drying time is 16 hours. Once dried, the support precursor is scraped from the filter paper using a spatula and transferred into a ceramic crucible. The crucible is placed into a laboratory chamber furnace and calcined. The preferred calcination temperature is 500°C. The calcination time at 500°C can vary, but the preferred value is 4 hours. The heating ramp to reach 500°C can vary between 1 and 20°C/minute, but the preferred value is 5°C/minute.

The active component of the catalyst is nickel in the form of nanoparticles, which are deposited over the support, in particular the ceria support. The term "nanoparticles" refers to particles of any shape with all three external dimensions in the nanoscale, i.e. in the range between 1 and 500 nm. Particle sizes in the nanometer range can be measured by transmission microscopy, or chemisorption techniques. Any amount of nickel can be used, but preferably between 1 and 10 wt. %. Any type of impregnation can be used, but the preferred method is using deposition precipitation. In preferred embodiments, the ceria nanorod powder is suspended in ultrapure water (conductivity of 18MΩ). The source of nickel can be any nickel containing salt, such as chloride, sulfate or acetate. Preferably, nickel nitrate is used for its easy solubility in water and no anion residue after calcination. The nickel salt is dissolved in ultrapure water at ambient temperature while stirring on a magnetic stirrer. The preferred stirring speed is 400-600 rpm. The support powder (preferably ceria) is added to the solution while stirring and the pH value of the suspension is increased to a value of 7.5 by aqueous ammonia solution. The pH value of the suspension is constantly monitored using a pH meter. After 2 hours of stirring, aqueous ammonia solution is added dropwise to raise the pH value to 9. Any concentration of ammonia solution can be used, but preferably 25 wt.% ammonia in water. After reaching pH value of 9, the slurry is left to mix for additional 30 minutes. After mixing, the suspension is filtered. The suspension is poured over a filter paper comprised of cellulose acetate and suction is provided by a vacuum pump. Once the filter cake is formed, it is washed continuously with about 500 ml of ultrapure water. The washed precursor on the filter paper is placed on the Petri dish and dried in the laboratory drier in air at 60°C. The preferred drying time is 16 hours. Once dried, the support precursor is scraped from the filter paper using a spatula and transferred into a ceramic crucible. The crucible is placed into a laboratory chamber furnace and calcined. The preferred calcination temperature is 500°C. The calcination time at 500°C can vary, but the preferred value is 4 hours. The heating ramp to reach 500°C can vary between 1 and 20°C/minute, but the preferred value is 5°C/minute.

Modification of the catalyst with alkali metals is crucial to improve ethane and ethylene selectivity. Any alkali metal can be used (Li, Na, K, Rb or Cs), but preferably potassium for ethane and caesium for ethylene. Typically, any alkali salt can be used as a source of alkali, such as sulfate, chloride or phosphate. Preferably, potassium hydroxide is used, which is dissolved in ultrapure water to produce 0.1 or 0.2 molar solutions. Between 0.2 and 2 wt.% alkali is deposited over the catalyst via impregnation. Preferably, incipient wetness impregnation method is used. The suitable amount of aqueous KOH solution which gives a desired 0.2-2 wt.% loading on the catalyst is used. The volume of KOH solution corresponds to the desired amount of alkali loading. The KOH solution is dripped over the catalyst powder, which is placed in a ceramic crucible. The impregnated catalyst precursor powder is mixed gently with a spatula to ensure homogeneous wetting of all powder.

For modification with caesium, any caesium salt can be used, such as sulfate, chloride or phosphate. Preferably, caesium hydroxide is used, which is dissolved in ultrapure water to produce 0.1 or 0.2 molar solutions. Any amount of alkali can be used, but preferably between 0.2 and 2 wt.%. Alkali is deposited over the catalyst via impregnation. Preferably, incipient wetness impregnation method is used. The suitable amount of aqueous CsOH solution which gives a desired 0.2-2 wt.% loading on the catalyst is used. The volume of CsOH solution corresponds to the desired alkali loading. The CsOH solution is dripped over the catalyst powder, which is placed in a ceramic crucible. The impregnated catalyst precursor powder is mixed gently with a spatula to ensure homogeneous wetting of all powder.

The ceramic crucible is placed in the laboratory drier for drying. Any temperature can be used for drying, but preferable drying is done at 60°C for 16 hours. After drying, the ceramic crucible is transferred to a chamber furnace. The catalyst precursor is calcined in air at 500°C for 4 hours. Any heating ramp between 1 and 20°C can be used to heat the furnace, at 5°C/minute is preferred. After the furnace cools naturally to ambient temperature, the catalyst is synthesized.

The synthesized catalyst powders enable catalytic hydrogenation of carbon dioxide to a gaseous mixture of ethane, ethylene, methane and carbon monoxide. Prior to catalytic reaction, the catalyst must be activated. That means it is transformed from the as-synthesized oxide form to partly reduced metallic form. This is achieved by heating the sample in hydrogen containing atmosphere. The hydrogen gas is mixed with an inert gas, such as helium, argon or nitrogen. Preferably, nitrogen is used. Hydrogen concentration can vary between 2 and 20 %. Preferably, 5% hydrogen in nitrogen is used for catalyst activation.

The catalyst activation is preferably done inside the catalytic reactor. This is preferably a fixed bed type of reactor, where the catalyst powder is placed in a form of a thin layer. The reactor is sealed and the activation gas flow (hydrogen diluted with nitrogen) in a top-down direction is initiated. The activation gas flow per amount of catalyst in the reactor is expressed as weight hourly space velocity (WHSV). The WHSV value provides the flowrate per mass of catalyst per unit time. Different WHSV values can be used, values between 1 and 10L/g_{catalyst}*minute. Preferably, a WHSV value of 2L/g_{catalyst}*minute is used. Once the catalyst is exposed to the activation flow, the temperature is increased to initiate activation. The activation takes place in the temperature range between 300 and 600°C. Preferably, activation takes place at 450°C. Different times can be used to complete activation, between 10 min and 2 hours. Preferably, 30 minutes of activation is used. The catalyst temperature is ramped between 2 and 20°C/minute. Preferably, catalyst is heated with a ramp of 10°C/minute.

Once the activation is over, the catalyst temperature is adjusted to values between 250 and 400°C. In this range of temperatures, the catalytic reaction of CO₂ hydrogenation occurs. The activation gas mixture (hydrogen diluted with nitrogen) is switched to a reactant flow mixture containing different amounts of CO₂, H₂ and inert. Inert can be any gas such as helium, argon or nitrogen. Preferably nitrogen is used. The concentration of inert can be between 1 and 25 %. Preferably, the concentration of inert is 15%. The concentrations of carbon dioxide and hydrogen together with inert accumulate to 100%. Any concentration of CO₂ between 10 and 60% can be used. Preferably, the CO₂ concentration is 25%. The hydrogen concentration must be in the range of 75 and 25%. Preferably, hydrogen concentration is 60%. The total flowrate of gas during the CO₂ hydrogenation reaction is expressed as WHSV. Any WHSV value between 1 and 10L/g_{catalyst}*minute can be used. Preferably, a GHSV value of 2L/g_{catalyst}*minute is used. The pressure inside the reactor can be anywhere between 1.1 and 23 barₐ. Preferably, 1.1 barₐ is used.

The invention is further illustrated by the following figures and examples. The catalyst samples are denoted as xKyNi-R or xCsyNi-R, where x represents mass percentage of alkali, K or Cs stand for potassium or caesium, y is mass percentage of nickel and R represents rod-like shape of cerium dioxide support.

### FIGURES

- **Fig. 1**: Ethane productivity as a function of temperature for different catalysts with a fixed amount of K (1.4 wt.%) and Ni (4 wt. %) on different supports: Ceria nanorods (R), commercial ceria powder (CeO₂ commercial) and silica gel (Davisil grade 646).
- **Fig. 2**: Ethane and ethylene productivity as a function of catalyst temperature for 6Ni-R catalysts (6wt.% Ni on ceria nanorods) containing the same molar amount of potassium or cesium.
- **Fig. 3**: Ethane, methane and CO productivity as a function of catalyst temperature for catalysts containing a fixed amount of potassium (1.4 wt. %) and an increasing amount of nickel (2-8 wt.%).
- **Fig. 4**: Ethane productivity as a function of catalyst temperature for 4Ni-R catalysts (4 wt.% nickel on ceria nanorods) modified with different amounts of potassium (0-2wt.%).
- **Fig. 5**: Ethylene productivity (**A**) and selectivity (**B**) for 4Ni-R and 6Ni-R catalysts modified with different amounts of Cs.

### EXAMPLES

### Example 1

This example demonstrates the crucial role of ceria nanorod support for enabling ethane productivity. Minimal or no ethane is produced when potassium modified nickel is deposited over other supports.

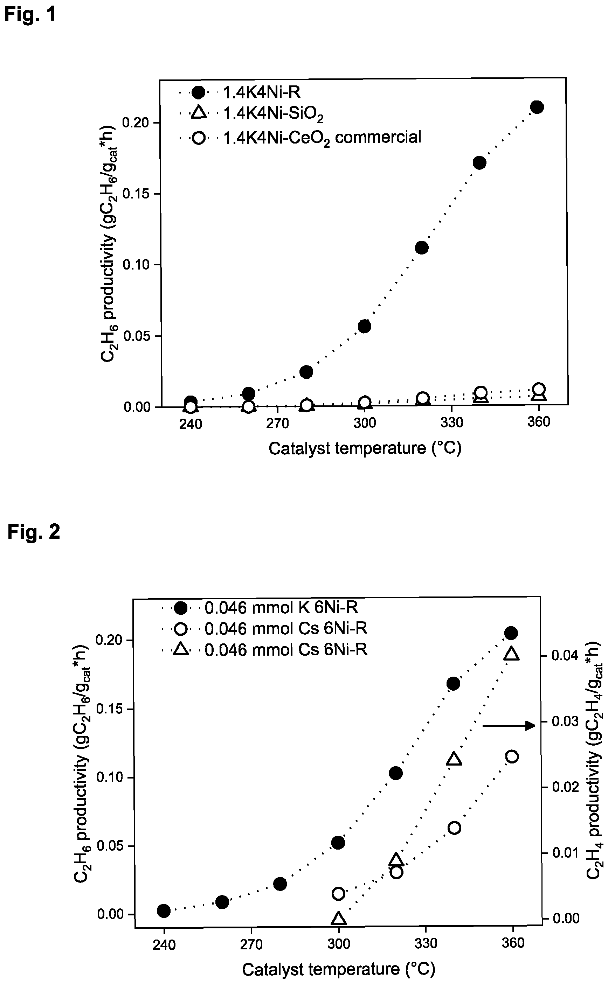

Ethane productivity at different temperatures is examined for different catalysts with a fixed amount of K (1.4 wt.%) and Ni (4 wt. %) on different supports: ceria nanorods (1.4K4Ni-R, 7mg), silica gel (1.4K4Ni-SiO₂) and commercial ceria powder (1.4K4Ni CeO₂ commercial, 15mg). Total flowrate= 15 ml/min, H₂:CO₂: N₂= 62:25:13. Prior to reaction, the catalyst was activated in 5% H₂/N₂ for 30 minutes at 450°C. The results are presented in Figure 1.

### Example 2

This example demonstrates the effect of alkali modification (K and Cs) on the ethane and ethylene productivity.

Ethane and ethylene productivity is examined at different catalyst temperatures for 6Ni-R catalysts (6wt.% Ni) containing the same molar amount of alkalis (potassium or cesium). Reaction conditions: 7 mg of catalyst, total flowrate= 15 ml/min, H₂:CO₂: N₂= 62:25:13. Prior to reaction, the catalyst was activated in 5% H₂/N₂ for 30 minutes at 450°C. The results are presented in Figure 2.

### Example 3

This example demonstrates the effect of different amounts of nickel and a fixed amount of potassium on the productivity of ethane, CO and methane.

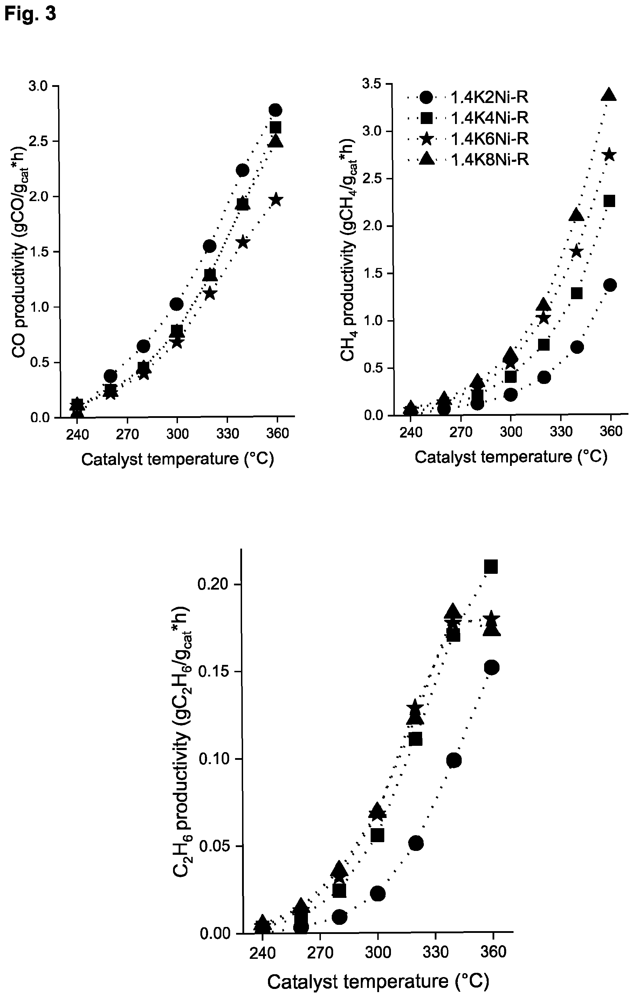

Ethane, methane and CO productivity is examined at different catalyst temperatures for catalysts containing a fixed amount of potassium (1.4 wt. %) and an increasing amount of nickel (2-8 wt.%) deposited over ceria nanorods. Reaction conditions: 7 mg of catalyst, total flowrate= 15 ml/min, H₂:CO₂: N₂= 62:25:13. Prior to reaction, the catalyst was activated in 5% H₂/N₂ for 30 minutes at 450°C. The results are presented in Figure 3.

### Example 4

This example demonstrates the effect of different potassium loading on ethane productivity for catalysts with a fixed nickel content.

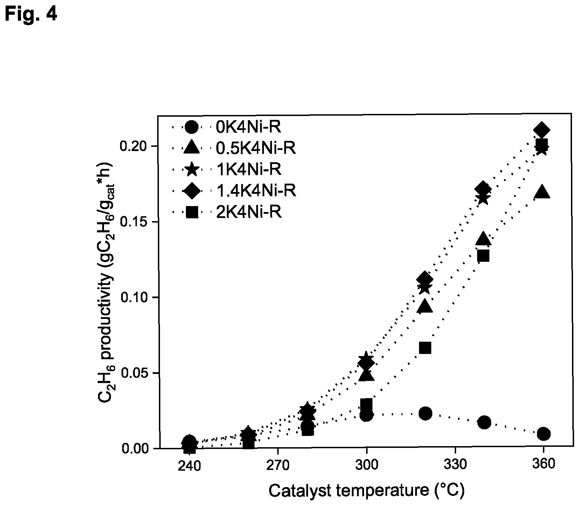

Ethane productivity is examined at different catalyst temperatures for 4Ni-R catalysts (4 wt.% nickel) on ceria nanorods, modified with different amounts of potassium (0-2wt.%). Reaction conditions: 7 mg of catalyst, total flowrate= 15 ml/minl, H₂:CO₂: N₂= 62:25:13. Prior to reaction, the catalyst was activated in 5% H₂/N₂ for 30 minutes at 450°C. The results are presented in Figure 4.

### Example 5

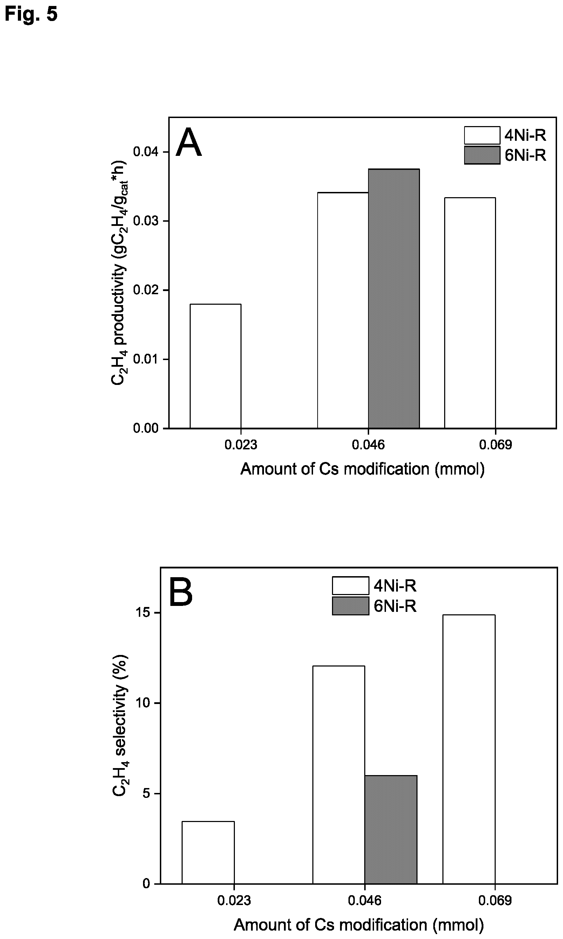

This example demonstrates the effect of different caesium loading on ethylene productivity (Figure 5A) and ethylene selectivity (Figure 5B) for catalysts with a 4 and 6 weight percent nickel content.

Ethylene productivity is examined at a constant catalyst temperature of 360°C. Catalysts (4 wt.% and 6 wt. %nickel) on ceria nanorods were modified with different amounts of caesium. Reaction conditions: 7 mg of catalyst, total flowrate= 15 ml/minl, H₂:CO₂: N₂= 62:25:13. Prior to reaction, the catalyst was activated in 5% H₂/N₂ for 30 minutes at 450°C. The results are presented in Figure 5.

## Claims

1. A process for direct hydrogenation of carbon dioxide gas, comprising the steps
(i) reacting carbon dioxide gas with molecular hydrogen in the presence of a heterogeneous catalyst comprising nickel nanoparticles on a solid support comprising CeO₂ nanorods, wherein the heterogeneous catalyst further comprises at least one alkali metal selected from K and Cs supported on the solid support, in particular wherein the amount of alkali metal is in a range of 0.2 to 2 wt.% based on the total weight of the catalyst; and
(ii) recovering gaseous reaction products comprising carbon monoxide, methane, and ethane and optionally ethylene.

2. The process according to claim 1, further comprising a step of isolating ethane and optionally additional C₂-hydrocarbons such as ethylene from the gaseous reaction products.

3. The process according to claim 1 or 2, wherein the heterogeneous catalyst comprises Ni nanoparticles in an amount of 1 to 10 wt.% based on the total weight of the catalyst.

4. The process according to any one of the preceding claims, wherein the solid support comprises a material in powder form, in particular a porous material containing pores with diameters in a range between 2 and 50 nm, in particular wherein the pore volume is in a range of about 0.05 to 0.6 cm³/g based on the total weight of the solid support and/or the specific surface area of the solid support is at least 5 m²/g, preferably in a range of 50 to 100 m²/g based on the total weight of the solid support.

5. The process according to any one of the preceding claims, wherein the solid support comprises a material selected from ceria, alumina, silica, and titania, preferably ceria.

6. The process according to any one of the preceding claims, wherein the CeO₂ nanorods have a diameter of about 10 to 20 nm and a length of about 100 to 500 nm.

7. The process according to any one of the preceding claims, wherein in step (i), a stream of a gaseous reactant mixture comprising or consisting of carbon dioxide, hydrogen and optionally an inert gas such as nitrogen, helium, argon or mixtures thereof, is contacted with the heterogeneous catalyst under heating, preferably to a temperature in a range of 250-400°C, more preferably 300-400°C, in particular about 360°C.

8. The process according to claim 7, wherein, in the reactant mixture, the concentration of hydrogen is in a range of 25-75% v/v, preferably about 60% v/v, the concentration of inert gas is in a range of 1-15 % v/v, preferably about 15% v/v, and the concentration of CO₂ is in a range of 10-60% v/v, preferably about 25% v/v.

9. The process according to claim 7 or 8, wherein the gas hourly speed velocity (GHSV) of the stream of the gaseous reactant mixture is in a range of 1-10L/g_{catalyst}*minute, preferably about 2L/g_{catalyst}*minute, and/or the pressure is in a range of 1.1-23 atmospheres.

10. The process according to any one of the preceding claims, wherein step (i) is carried out in a fixed bed reactor, preferably comprising a thin layer of the heterogeneous catalyst in powder form.

11. The process according to any one of the preceding claims comprising a preceding step of activating the heterogeneous catalyst before step (i), preferably by heating the heterogeneous catalyst in a hydrogen-containing atmosphere to a temperature in a range of 300-600°C, in particular 400-500°C, e.g. about 450°C.

12. The process according to claim 11, wherein activating the heterogeneous catalyst comprises exposing the catalyst to an activation gas stream comprising hydrogen in a concentration of about 2-20% v/v, preferably about 5% v/v, and an inert gas, in particular nitrogen, helium, argon or mixtures thereof, preferably nitrogen, under heating, wherein the gas hourly space velocity (GHSV) of the gas stream preferably is in a range of 1 to 10L/g_{catalyst}*minute, more preferably about 2L/g_{catalyst}*minute, and/or wherein the activation is carried out for a term of 10 min to 2 h, preferably about 30 min, and/or wherein heating comprises ramping the temperature in a range of 2 and 20°C/minute, preferably with a ramp of 10°C/minute.
